# EUROPEAN PATENT SPECIFICATION

(11) **EP 3 187 175 B1**
(45) Date of publication and mention of the grant of the patent: **31.03.2021**
(21) Application number: 16206400.0
(22) Date of filing: 22.12.2016
(51) Int. Cl.: A61K 31/19, A61K 9/20, A61K 9/48, A61K 35/745, A61K 9/16, A61K 9/28, A61P 1/00, A61K 36/752

(54) **SUPPLEMENTARY THERAPEUTIC FORMULATIONS FOR THE SEQUENTIAL OR SEPARATE ADMINISTRATION OF BUTYRIC ACID AND PROBIOTICS**
ZUSÄTZLICHE THERAPEUTISCHE FORMULIERUNGEN ZUR SEQUENZIELLEN ODER SEPARATEN VERABREICHUNG VON BUTTERSÄURE UND PROBIOTIKA
FORMULATIONS THÉRAPEUTIQUES SUPPLÉMENTAIRES POUR L'ADMINISTRATION SÉQUENTIELLE OU SÉPARÉE D'ACIDE BUTYRIQUE ET PROBIOTIQUES

(30) Priority: 22.12.2015 IT UB20159138
(43) Date of publication of application: 05.07.2017
(73) Proprietor: Euro-Pharma S.r.l., 10127 Torino (IT)
(72) Inventor: MISCIOSCIA, Mario, 10127 TORINO (IT)
(74) Representative: Coppo, Alessandro

(56) References cited:
- EP-A1- 1 236 466
- EP-A1- 2 289 505
- WO-A1-2005/074718
- WO-A1-2007/125558
- WO-A1-2012/013495
- WO-A1-2014/152338
- LEE TAE-HO ET AL: "The Inhibitory Effect of Grapefruit Seed Extracts on the Physiological Function of Enterobacter pyrinus", KOREAN JOURNAL OF FOOD SCIENCE AND TECHNOLOGY, SEOUL, KR, vol. 27, no. 6, 31 December 1995 (1995-12-31), pages 985-990, XP053022952, ISSN: 0367-6293

## Description

### Field of the invention

The present invention relates to the field of supplementary formulations for special medical purposes, in particular that containing butyric acid, probiotics, and prebiotics.

### Background art

In the field of normal food supplements, the use of lactic acid bacteria has been widespread for many years, which have the effect of promoting the balance of intestinal flora with both a preventive effect (keeping a healthy and efficient bacterial flora), and with an implementing effect, if it is necessary to enrich the bacterial flora following the administration of antibiotics or in the presence of typical intestinal disorders, such as: IBS (irritable bowel syndrome, diarrhea, meteorism, flatulence, etc.), inflammatory colitis, infectious colitis, ulcerative colitis, diverticulitis, IBD (Crohn's disease).

Said probiotics are normally associated with substance called prebiotics which as food allow the growth and the development of probiotics.

Moreover, more recently it has been found that butyric acid, or salts or esters thereof having anti-inflammatory activity, is capable of facilitating the beneficial effect of probiotics in the intestinal duct and preparations have thus become widespread which contain these three components at the same time, and not always: butyric acid or salts or esters thereof, probiotics and prebiotics.

EP2289505 describes some of these preparations, having gastro-resistant properties.

While these formulations are generally considered useful, they are unsuitable for a specific supplementary use for the functional and healthy restoration as regards individual and specific interactive requirements related to individual pathological conditions of the intestinal tract and, specifically, of the colon.

In fact in certain cases, the administration of probiotics is useless since, for example, it is nullified by the intake of antibiotics which obviously kill them, or also by a significant and invasive inflammatory aspect which, by destroying the mucosal colon layer, does not allow the engraftment thereof, making them unusable.

For example, in the case of infectious colitis, an antibiotic therapy is required with the concurrent association of an anti-inflammatory therapy, which must be followed by a probiotic therapy, which if taken simultaneously, is nullified by the antibiotic.

In the case of ulcerative colitis, a disease with high infectious, inflammatory and mucosa-damaging impact where butyric acid, due to its specific anti-inflammatory and mucosa-repairing action, is also a valid contribution in the context of an antibiotic therapy; if probiotics are taken simultaneously, these are inactive since the severe inflammation and mucosa-damaging condition and the presence of antibiotic therapy would nullify the adhesion, the vitality and thus the action thereof. It is therefore essential to first intervene against the infectious, inflammatory and reparative aspect and then provide to restoring the targeted bacterial flora by taking the specific probiotic strains.

The irritable bowel syndrome (IBS) or inflammatory colitis appears with a colon inflammatory syndrome with diarrhea and meteorism events. In this case, an anti-inflammatory therapy is administered first as attack method and, generally after twelve hours, probiotics with a targeted anti-inflammatory and immune action plus prebiotics.

Diverticulitis is a disease with significant harmful and inflammatory impact on the large intestine and colon, with the formation of loops which frequently become diverticula. For these problems, a supplemented antibiotic and anti-inflammatory therapy is first required, followed by the intake of probiotic plus prebiotics adapted to the immune restoration and re-colonization of the intestinal flora.

In the light of the above, the need of having formulations which are more efficient and suitable for the administration in the various different situations in which the subject active ingredients may be useful is therefore clear.

### Summary of the invention

The present invention solves the above problem with a kit for the separate or sequential administration of butyric acid or salts or esters thereof, preferably in combination with prebiotics.

Advantageously, in the kit of the invention, butyric acid, optionally in the form of a salt or ester, is formulated or contained in a slow-release and gastro-resistant tablet, probiotics and prebiotics are formulated or contained in a gastro-resistant capsule. According to some embodiments, the probiotics are microencapsulated and preferably contained in a capsule with prebiotics. Butyric acid salts, according to the invention, are both organic and inorganic salts, particularly preferred are calcium and sodium salts.

Probiotics are all those microorganisms capable of stabilizing the intestinal flora, such as those belonging to the genera: *Bifidobacterium, Lactobacillus and Streptococcus.*

Among the above microorganisms, the following are particularly preferred:
*Lactobacillus plantarum, Lactobacillus reuteri, Bifidobacterium brevis.*

According to embodiments of the invention, the probiotics are included in capsules so as to prevent damage and even death caused by the compression to make the tablet.

Prebiotics typically are fructooligosaccharides (FOS) and galactooligosaccharides (GOS), where the former are preferred.

According to an aspect, the invention relates to a kit comprising sustained-release, gastro-resistant tablets comprising butyric acid or salts or esters thereof, possibly in combination with products, either natural or synthetic, having activity against intestinal disorders, and gastro-resistant capsules consisting of microencapsulated probiotics in combination with prebiotics and advantageously according to any one of the embodiments of claims 2-14.

### Detailed description of the invention

The kit according to some embodiments of the present invention therefore comprises a capsule containing one or more probiotic preferably selected from the group consisting of: *Lactobacillus plantarum, Lactobacillus reuteri, Bifidobacterium brevis,* preferably in combination with one or more prebiotics, and an advantageously gastro-resistant and sustained-release tablet, containing butyric acid or salts or esters thereof.

According to preferred embodiments, a sequential administration of the components of the inventive kit is provided, comprising a first step of administration of the tablet containing butyric acid and a second step of administration of the capsule containing probiotics, preferably in combination with prebiotics.

Typically, the administration regimen provides that at least two, three, four, five, six, seven, eight, nine, ten days elapse between the administration of tablets and capsules.

The separate or sequential administration of butyric acid allows to reduce the inflammation of the colonic mucosa of the intestine and prepare the mucosa to the treatment and colonization of the colon with probiotics, in particular when co-administered with prebiotics. The Applicant has found that the sequential treatment of the invention can significantly reduce the inflammation of the mucous membranes and intestines walls and reduce the recovery time of physiological activities, with a reduction of relapse.

The tablet containing butyric acid or derivatives thereof as defined is preferably a gastro-resistant sustained-release tablet.

If preferred, the above tablet containing butyric acid may also comprise other products, either natural or synthetic, active in the prevention or treatment of conditions mentioned, above such as for example: G.S.E., Aloe, Lapacho, Curcumin, MSM (methylsulfonylmethane).

According to a particular embodiment of the invention, the tablet contains Grapefruit Seed Extract (G.S.E.), a natural compound, typically in the form of powder, which is defined "the most powerful natural antibiotic" against bacteria, both Gram-positive and Gram-negative, in addition to having other important activities: (anti-fungal and anti-inflammatory, etc.) without altering the intestinal flora.

The presence of G.S.E. in the sustained-release tablet therefore allows to associate the anti-inflammatory and reparative action of butyric acid with a significant antibacterial, anti-inflammatory and also antifungal added value, without adding any significant interaction with other concurrent therapies and also without any side effects; an action which is particularly important for example in the case of ulcerative colitis or in the case of IBS, where the association of Butyric Acid and G.S.E. in the tablet represents a valuable contribution in helping to treat IBS, since in some cases, infectious events may occur and in such cases, the antibacterial action of G.S.E., which does not adversely interact with probiotics(-), is considered to be of great help.

Both the capsule and the tablet may then contain other components, such as retardants such as HPMC, excipients such as microcrystalline cellulose, magnesium stearate, etc.

Typically, for the tablet to be gastro-resistant, may be coated with a shellac, glycerol and talc film, or with a modified starch, glycerol, talc and titanium dioxide film, or with a Eudraguard, triethyl citrate and talc film.

Preferably, the individual probiotics are contained in the capsule in an amount of between 5x10⁶ - 30x10⁹, more preferably of between 200x10⁶ - 1x10⁹ with microencapsulation technique.

Butyric Acid may typically be present, in the case of sustained-release tablets, in an amount of between 100 mg and 1000 mg, preferably 200 and 600 mg, while the prebiotics are present in amounts of between 100 mg and 2 g, more preferably of between 200 mg and 1 g.

The other active ingredients, such as the above G.S.E., if present, are in amounts of between 20 mg and 900 mg, more preferably of between 40 mg and 200 mg. The tablets and capsules according to the invention are prepared using pharmaceutical technology processes.

For example, in the case of tablets, the components are mixed in a mixer in which the association with HPMC produces the sustained release in the core, containing Butyric Acid, when reaching the Colon, where it starts to dissolve at a pH greater than 6.5, and in particular in formula at pH 6.8. The resulting mixture is then compressed using a tablet press; the tablets thus obtained are then coated with the gastro-resistant layer in a coating pan until obtaining the desired thickness, after which the resulting tablets are blistered.

Likewise, the capsules may be produced starting from the freeze-drying cake of probiotics, which is subjected to granulation to obtain a uniform particle size, and then standardizing the final granulate.

The granules are then microencapsulated according to known processes (see for example WO 2013/114185) which use a fluid bed equipped for the coating process of the bacterial cells, for example using plant lipids (such as mono- and diglycerides of fatty acids) which coat the bacterial cell, thereby imparting the desired gastro-resistance.

The microcapsules are then encapsulated in vegetable capsules, for example consisting of HPMC, and then blistered.

Below are some examples of the invention.

### EXAMPLE 1

### Preparation of the capsules

The freeze-drying cakes of Lactobacillus plantarum LP021, Lactobacillus reuteri LRE02 and Bifidobacterium brevis BR03 are granulated so as to break and standardize the cakes to obtain a uniform particle size.

At the end of the granulation process, the granulate is standardized with inulin to a charge of 100 MLD CFU/g.

The individual freeze-dried and granulated strains are then microencapsulated using lipids of plant origin.

At the end of the microencapsulation process, the microencapsulated and thus gastro-protected probiotic cells are then mixed as follows (percentages are by weight on the total weight):

| | |
|---|---|
| Lactobacillus plantarum LP01 | 7% |
| Lactobacillus reuteri LRE02 | 1.5% |
| Bifidobecterium brevis BR03 | 7% |
| Fructo-oligosaccharide | 84.5% |

so a mixture portion as above, which is encapsulated, consists of:

| | |
|---|---|
| Lactobacillus plantarum LP01 | 19.6 mg |
| Lactobacillus reuteri LRE02 | 4.2 mg |
| Bifidobecterium brevis BR03 | 19.6 mg |
| Fructo-oligosaccharide | 236.6 mg |

with a total weight of 280 mg powder.

At the end of the mixing process, the semi-finished product is encapsulated in vegetable capsules consisting of HPMC, which are then blistered with OPA (PA 25MY/ALL 45 MY/PVC 60 MY) and 25 micron aluminum.

Each capsule has a weight of about 335 mg.

### EXAMPLE 2

### Preparation of the tablets

The raw materials to be mixed in the order given are added to a mixer:

| | |
|---|---|
| Calcium butyrate (first part) | 30.7 kg |
| G.S.E. | 5.0 kg |
| Silicon dioxide | 0.3 kg |
| Hydroxypropylmethylcellulose (HPMC) | 15.0 kg |
| Microcrystalline cellulose | 16.3 kg |
| Calcium butyrate (second part) | 30.7 kg |
| Mono- and diglycerides of fatty acids | 1.7 kg |

and then mixing is carried out for 20 minutes. 0.3 kg of vegetable magnesium stearate are then added to the mixture, continuing the mixing for another 5'.

The resulting mixture is compressed using a tablet press set up with the following parameters:

| | |
|---|---|
| Tablet size: | 19 x 8.8 mm |
| Tablet weight: | 1.000 gram |

100 kg of non-filmed tablets are obtained.

The film-forming solution is then prepared by adding the following three alternatives (alternative examples A, B, C) of raw materials to a dissolver in the order given:
A)

| | |
|---|---|
| Shellac | 0.6245 kg |
| Glycerol | 0.0626 kg |
| Talc | 0.3129 kg |
| | Total in the mixture 1 kg |

B)

| | |
|---|---|
| Modified starch (E1420) | |
| Glycerol F.U. | |
| Talc | |
| Titanium dioxide | |
| | Total in the mixture 25 kg/100 kg |

C)

| | |
|---|---|
| Eudraguard E 1207 | |
| Triethyl citrate (E1505) | |
| Talc | |
| | Total in the mixture 5 kg/100 kg |

In all three cases A-C, demineralized water as needed is added to dissolution. The resulting film-forming solution is sprayed onto the non-filmed tablets previously obtained in Example A using a coating pan, to obtain a coated tablet weighing about 1010 mg; in total, about 101 kg of filmed tablets are obtained.

In the case of the above example B, the weight of the coated tablet is 1250 mg; in total, about 125 kg of filmed tablets are obtained.

In the case of the above example C, the weight of the coated tablet is 1050 mg; in total, about 105 kg of filmed tablets are obtained.

Both the filmed tablets and the capsules are normally packaged in the form of a blister of different color (such as blue and red) which are then boxed so that each box contains both the capsule blister and the tablet blister.

Typically, each box will contain 5 to 60 capsules and 5 to 60 tablets.

The kits according to the invention therefore allow to utilize the synergistic but at the same time separate action of butyric acid (and any other possible functional principles, such as G.S.E.), specific probiotics and FOS, after reaching the colon, allowing the physician to customize and target the therapy, diversifying it according to every single situation.

## Claims

1. A kit comprising gastro-resistant, sustained-release tablets comprising butyric acid or salts or esters thereof and gastro-resistant capsules comprising probiotics optionally in combination with prebiotics.

2. A kit according to claim 1, wherein said butyric acid salts are organic or inorganic salts, particularly calcium or sodium salts.

3. A kit according to claim 1 or 2, wherein said probiotics are selected from *Lactobacillus plantarum, Lactobacillus reuteri, Bifidobacterium brevis* and mixtures thereof, preferably in amounts of between 5x10⁶-30x10⁹ and the prebiotics are selected from fructooligosaccharides and galactooligosaccharides and mixtures thereof, preferably in amounts of between 100 mg and 2 g.

4. A kit according to claims 1 - 3, wherein said probiotics are microencapsulated.

5. A kit according to any one of claims 1 - 4, further comprising one or more products for the treatment of intestinal diseases or disorders.

6. A kit according to claim 5, wherein said products for the treatment of intestinal diseases or disorders are selected from: Grapefruit Seed Extract, Aloe, Lapacho, Curcumin, methylsulfonylmethane and mixtures thereof, preferably in amounts of between 20 mg and 900 mg.

7. A kit according to any one of claims 1 - 6, wherein the gastro-resistant tablet comprises an outer filming and an sustained-release core containing butyric acid and Grapefruit Seed Extract

8. A kit according to claims 1-7, consisting of:
Tablets consisting of:
| | |
|---|---|
| Calcium butyrate (first part) | 614 mg |
| Grapefruit Seed Extract (G.S.E.) | 50 mg |
| Hydroxypropylmethylcellulose (HPMC) | 150 mg |
| Microcrystalline cellulose | 163 mg |
| Mono and diglycerides | 17 mg |
| Silicon dioxide | 19 mg |
| vegetable magnesium stearate | 3 mg |
with a filming consisting of:
| | |
|---|---|
| Shellac | 6.245 mg |
| Glycerol | 0.626 mg |
| Talc | 3.129 mg |
| Demineralized water | as needed for dissolution |
| total weight | 1010 mg |
and
Capsules consisting of:
| | |
|---|---|
| Lactobacillus plantarum LP01 | 19.6 mg |
| Lactobacillus reuteri LRE02 | 4.2 mg |
| Bifidobecterium brevis BR03 | 19.6 mg |
| Fructooligosaccharides | 236.6 mg |
total weight 280 mg of powder encapsulated in vegetable capsules consisting of HPMC with a total weight of the capsule of 355 mg.

9. A kit according to claims 1-8, containing 5-60 capsule blisters and 5-60 tablet blisters.

10. A kit according to any one of claims 1-9, for use in the treatment of intestinal diseases or disorders, in particular the colon.

11. A kit for use according to claim 10, wherein said intestinal diseases or disorders are irritable bowel syndrome, inflammatory colitis, infectious colitis, ulcerative colitis, diverticulitis or Crohn's disease.

12. A kit for use according to claim 10 or 11, for the sequential or separate administration of
- gastro-resistant, sustained-release tablets comprising butyric acid or salts or esters thereof, and
- gastro-resistant capsules comprising probiotics optionally in combination with prebiotics.

13. A kit for use according to claim 12, wherein said gastro-resistant and sustained-release tablets contain Grapefruit Seed Extract.

14. A gastro-resistant delayed-release tablet consisting of butyric acid and Grapefruit Seed Extract.

## Patentansprüche

1. Kit, umfassend magensaftresistente Retardtabletten, umfassend Buttersäure oder Salze oder Ester davon und magensaftresistente Kapseln, umfassend Probiotika, gegebenenfalls in Kombination mit Präbiotika.

2. Kit nach Anspruch 1, wobei die Buttersäuresalze organische oder anorganische Salze sind, insbesondere Kalzium- oder Natriumsalze.

3. Kit nach Anspruch 1 oder 2, wobei die Probiotika aus *Lactobacillus plantarum, Lactobacillus reuteri, Bifidobacterium brevis* und Gemischen davon ausgewählt sind, vorzugsweise in Mengen zwischen 5x10⁶-30x10⁹, und die Präbiotika aus Fructooligosacchariden und Galactooligosacchariden und Gemischen davon ausgewählt sind, vorzugsweise zwischen 100 mg und 2 g.

4. Kit nach den Ansprüchen 1-3, wobei die Probiotika mikroverkapselt sind.

5. Kit nach irgendeinem der Ansprüche 1-4, ferner umfassend ein oder mehrere Produkte zur Behandlung von Darmerkrankungen oder --störungen.

6. Kit nach Anspruch 5, wobei die Produkte zur Behandlung von Darmerkrankungen oder --störungen ausgewählt sind aus:
Grapefruitsamenextrakt, Aloe, Lapacho, Curcumin, Methylsulfonylmethan und
Gemischen davon, vorzugsweise in Mengen zwischen 20 mg und 900 mg.

7. Kit nach irgendeinem der Ansprüche 1-6, wobei die magensaftresistente Tablette eine Außenbefilmung und einen Retardkern umfasst, der Buttersäure und Grapefruitsamenextrakt enthält.

8. Kit nach den Ansprüchen 1-7, bestehend aus:
Tabletten bestehend aus:
| | |
|---|---|
| Calciumbutyrat (erster Teil) | 614 mg |
| Grapefruitsamenextrakt (G.S.E.) | 50 mg |
| Hydroxypropylmethylcellulose (HPMC) | 150 mg |
| Mikrokristalline Cellulose | 163 mg |
| Mono- und Diglyceride | 17 mg |
| Siliciumdioxid | 19 mg |
| pflanzliches Magnesiumstearat | 3 mg |
mit einer Befilmung bestehend aus:
| | |
|---|---|
| Schellack | 6.245 mg |
| Glycerin | 0.626 mg |
| Talk | 3.129 mg |
| Demineralisiertes Wasser | nach Bedarf für die Auflösung |
| Gesamtgewicht | 1010 mg |
und
Kapseln bestehend aus:
| | |
|---|---|
| Lactobacillus plantarum LP01 | 19.6 mg |
| Lactobacillus reuteri LRE02 | 4.2 mg |
| Bifidobecterium brevis BR03 | 19.6 mg |
| Fructooligosaccharide | 236.6 mg |
Gesamtgewicht 280 mg Pulver, eingekapselt in pflanzliche Kapseln, bestehend aus HPMC mit einem Gesamtgewicht der Kapsel von 355 mg.

9. Kit nach den Ansprüchen 1-8, das 5-60 Kapselblister und 5-60 Tablettenblister enthält.

10. Kit nach irgendeinem der Ansprüche 1-9 zur Verwendung bei der Behandlung von Darmerkrankungen oder -störungen, insbesondere des Dickdarms.

11. Kit zur Verwendung nach Anspruch 10, wobei die Darmerkrankungen oder --störungen Reizdarmsyndrom, entzündliche Kolitis, infektiöse Kolitis, Colitis ulcerosa, Divertikulitis oder Morbus Crohn sind.

12. Kit zur Verwendung nach Anspruch 10 oder 11 zur sequentiellen oder getrennten Verabreichung von
- magensaftresistente Retardtabletten, die Buttersäure oder Salze oder Ester davon umfassen, und
- magensaftresistente Kapseln umfassend Probiotika, gegebenenfalls in Kombination mit Präbiotika.

13. Kit zur Verwendung nach Anspruch 12, wobei die magensaftresistenten und Retardtabletten Grapefruitsamenextrakt enthalten.

14. Magensaftresistente Retardtablette bestehend aus Buttersäure und Grapefruitsamenextrakt.

## Revendications

1. Kit comprenant des pastilles gastro-résistantes à libération prolongée comprenant de l'acide butyrique ou des sels ou des esters de celui-ci et des capsules gastro-résistantes comprenant des probiotiques facultativement en combinaison avec des prébiotiques.

2. Kit selon la revendication 1, dans lequel lesdits sels d'acide butyrique sont des sels organiques ou inorganiques, en particulier des sels de calcium ou de sodium.

3. Kit selon la revendication 1 ou 2, dans lequel lesdits probiotiques sont sélectionnés parmi *Lactobacillus plantarum, Lactobacillus reuteri, Bifidobacterium brevis* et leurs mélanges, de préférence en quantités entre 5x10⁶-30x10⁹ et les prébiotiques sont sélectionnés parmi les fructo-oligosaccharides et les galacto-oligosaccharides et leurs mélanges, de préférence en quantités comprises entre 100 mg et 2 g.

4. Kit selon les revendications 1-3, dans lequel lesdits probiotiques sont microencapsulés.

5. Kit selon l'une quelconque des revendications 1-4, comprenant en outre un ou plusieurs produits pour le traitement de maladies ou troubles intestinaux.

6. Kit selon la revendication 5, dans lequel lesdits produits pour le traitement de maladies ou troubles intestinaux sont sélectionnés parmi : extrait de pépins de pamplemousse, aloès, lapacho, curcumine, méthylsulfonylméthane et leurs mélanges, de préférence en quantités comprises entre 20 mg et 900 mg.

7. Kit selon l'une quelconque des revendications 1-6, dans lequel la pastille gastro-résistante comprend un enrobage externe et un noyau à libération prolongée contenant de l'acide butyrique et de l'extrait de pépins de pamplemousse

8. Kit selon les revendications 1-7, constitué de :
Pastilles constituées de :
| | |
|---|---|
| Butyrate de calcium (première partie) | 614 mg |
| Extrait de pépins de pamplemousse (E.P.P.) | 50 mg |
| Hydroxypropylméthylcellulose (HPMC) | 150 mg |
| Cellulose microcristalline | 163 mg |
| Mono et diglycérides | 17 mg |
| Dioxyde de silicium | 19 mg |
| stéarate de magnésium végétal | 3 mg |
avec un enrobage constitué de :
| | |
|---|---|
| Gomme-laque | 6,245 mg |
| Glycérol | 0,626 mg |
| Talc | 3,129 mg |
| Eau déminéralisée | assez pour la dissolution |
| poids total | 1010 mg |
et
Capsules constituées de :
| | |
|---|---|
| Lactobacillus plantarum LP01 | 19,6 mg |
| Lactobacillus reuteri LRE02 | 4,2 mg |
| Bifidobacterium brevis BR03 | 19,6 mg |
| Fructo-oligosaccharides | 236,6 mg |
poids total 280 mg de poudre encapsulée dans des capsules végétales constituées de HPMC avec un poids total de la capsule de 355 mg.

9. Kit selon les revendications 1-8 contenant 5-60 blisters de capsules et 5-60 blisters de pastilles.

10. Kit selon l'une quelconque des revendications 1-9, pour une utilisation dans le traitement de maladies ou troubles intestinaux, en particulier du colon.

11. Kit pour une utilisation selon la revendication 10, dans lequel lesdites maladies ou troubles intestinaux sont le syndrome de l'intestin irritable, la colite inflammatoire, la colite infectieuse, la colite ulcéreuse, la diverticulite ou la maladie de Crohn.

12. Kit pour une utilisation selon la revendication 10 ou 11, pour l'administration séquentielle ou séparée de
- pastilles gastro-résistantes à libération prolongée comprenant de l'acide butyrique ou des sels ou des esters de celui-ci, et
- capsules gastro-résistantes comprenant des probiotiques facultativement en combinaison avec des prébiotiques.

13. Kit pour une utilisation selon la revendication 12, dans lequel lesdites pastilles gastro-résistantes et à libération prolongée contiennent de l'extrait de pépins de pamplemousse.

14. Pastille gastro-résistante à libération retardée constituée d'acide butyrique et extrait de pépins de pamplemousse.
